# EUROPÄISCHE PATENTSCHRIFT

(11) **EP 1 586 276 B1**
(45) Veröffentlichungstag und Bekanntmachung des Hinweises auf die Patenterteilung: **25.11.2009**
(21) Anmeldenummer: 05008060.5
(22) Anmeldetag: 13.04.2005
(51) Int. Cl.: A61B 17/68

(54) **Stabilisierungseinrichtung für Knochen und Herstellungsverfahren für ein elastisches Element**
Stabilising device for bones and method of fabrication of an elastic element
Dispositif de stabilisation pour des os et la méthode de fabrication d'un élément élastique

(30) Priorität: 16.04.2004 DE 102004018621; 16.04.2004 US 563241 P
(43) Veröffentlichungstag der Anmeldung: 19.10.2005
(73) Patentinhaber: BIEDERMANN MOTECH GmbH, 78054 VS-Schwenningen (DE)
(72) Erfinder: Harms, Jürgen, 76227 Karlsruhe (DE); Biedermann, Lutz, 78048 VS-Villingen (DE); Matthis, Wilfried, 79367 Weisweil (DE)
(74) Vertreter: Hofer, Dorothea

(56) Entgegenhaltungen:
- WO-A-03/047442
- WO-A-2004/105577
- US-A- 5 306 310
- US-A- 5 423 816
- US-A- 5 500 122
- US-A- 5 944 302
- US-A- 6 162 223
- US-B1- 6 197 065
- US-B1- 6 656 184

## Beschreibung

Die Erfindung betrifft eine Stabilisierungseinrichtung für Knochen oder Wirbel mit einem elastischen Element

Zur Fixierung von Knochenfrakturen oder zur Stabilisierung der Wirbelsäule sind Fixations- und Stabilisierungseinrichtungen bekannt, die aus wenigstens zwei im Knochen bzw. Wirbel verankerten und über eine Platte oder über einem Stab verbundenen Knochenschrauben bestehen. Derartige starre Systeme erlauben keine Bewegung der relativ zueinander fixierten Knochenteile oder Wirbel.

Für bestimmte Indikationen ist jedoch eine dynamische Stabilisierung wünschenswert, bei der die zu stabilisierenden Knochenteile und Wirbel eine kontrollierte begrenzte Bewegung zu einander ausführen können. Eine Möglichkeit für die Realisierung der dynamischen Stabilisierungseinrichtung besteht in der Verwendung eines elastischen Elementes anstelle eines die Knochenverankerungselemente verbindenden starren Stabes.

Aus der US 2003/0109880 A1 ist eine dynamische Stabilisierungseinrichtung für Wirbel bekannt, die eine erste und eine zweite im Wirbel zu verankernde Schraube jeweils mit einem Aufnahmeteil zum Einlegen einer die Schrauben verbindenden Feder und eine solche Feder umfaßt. Die Feder selbst ist als Ganzes in Form einer Schraubenfeder mit dicht benachbarten Windungen nach Art einer Zugfeder ausgebildet und wird über Klemmschrauben in den Aufnahmeteilen fixiert. Es besteht hierbei jedoch die Gefahr, dass die Feder auf Grund ihrer Elastizität dem Druck der Klemmschraube ausweicht und somit die Fixierung zwischen der Knochenschraube und der Feder gelockert wird. Ein weiterer Nachteil der Vorrichtung besteht darin, dass die Elastizität der Feder bei ansonsten gleichen Federeigenschaften von der Länge der Feder abhängt. Ferner ist die Biegesteifigkeit der Feder insbesondere bei kurzen Baulängen relativ gering.

Aus der US 6,162,223 ist eine Fixationseinrichtung für ein Gelenk, z.B. für ein Handgelenk oder ein Kniegelenk, bekannt, bei der ein an seinen Enden mit Knochenverankerungselementen verbundener Fixationsstab zweiteilig ausgebildet ist, wobei die zwei Teile des Fixationsstabs über ein flexibles Kupplungsteil miteinander verbunden sind und wobei die Fixationsstäbe um das Kupplungsteil außerhalb des Körpers angebracht sind. Die beiden Teile des Fixationsstabs sind mit dem Kupplungsteil nicht fest verbunden, sondern können sich entlang einer Bohrung in dem Kupplungsteil frei bewegen. Der Durchmesser des Kupplungsteil ist auf Grund der Art der Verbindung mit dem zweiteiligen Fixationsstab immer größer als der Durchmesser des Fixationsstabs. Diese bekannte Fixationseinrichtung ist auf Grund ihres komplizierten und voluminösen Aufbaus zum körperinternen Einsatz an der Wirbelsäule oder anderen Knochen nicht geeignet. Insbesondere die Realisierung eines solchen flexiblen Kupplungsteils mit hoher Biegesteifigkeit erfordert ein großes Bauvolumen

US 5,423,816 offenbart eine Zwischenwirbelverriegelungsvorrichtung mit einem spiralförmigen elastischen Körper, zwei Befestigungslaschen und zwei Verriegelungselementen. Knochenspäne umgeben das elastische Element und die Kammern des elastischen Elements.

US 6,197,065 beschreibt eine Knochenbefestigungsanordnung für den verbleibenden Abschnitt einer Röhrenknochendiaphyse. Die Anordnung weist einen Hauptkörper auf, einen Anker zum Verankern der Vorrichtung bezüglich des verbleibenden Knochenabschnitts und einen Befestigungsabschnitt zum Befestigen des Hauptkörper an dem Anker. Der Befestigungsabschnitt weist einen elastischen Abschnitt auf, der als-Schraubenfeder ausgebildet ist.

Die altere An-meldung WO 2004/105577 A2 beschreibt ein Wirbalsäulenstabilisierungssystem mit einem oder mehreren flexiblen Elementen mit einer Öffnung oder einem Schlitz. Das flexible Element ist einstückig in einem Stab ausgebildet, der Enden aufweist, die Befestigungsschrauben aufnehmen können. In einem Ausführungsbeispiel sind zwei Schlitze oder Ausnehmungen in dem Stab ähnlich zu einer Doppelhelix gebildet. Dieses Dockument ist stand der Technik nach Artikel 54(3) EPÜ.

Es ist Aufgabe der Erfindung, eine Stabilisierungsvorrichtug für Knochen oder Wirbel mit einem elastischen Element bereit zu stellen, das eine hohe Biegesteifigkeit bei geringer Baulänge hat, sowie leicht zu handhaben ist bei gleichzeitig hoher Sicherheit im Einsatz, und welches mit anderen Elementen in möglichst vielfältiger Weise zu einer dynamischen Stabilisierungseinrichtung für Wirbel oder Knochen kombiniert werden kann.

Die Aufgabe wird gelöst durch eine Stabilisierungsvorrichtung nach dem Patentanspruch 1.

Weiterbildungen der Erfindung sind in den Unteransprüchen angegeben.

Die Erfindung weist den Vorteil auf, dass ein elastisches Element zugleich kompakt und mit hoher Biegesteifigkeit ausgebildet ist. Dies ist insbesondere für Anwendungen an der Wirbelsäule und dort insbesondere an der Halswirbelsäule von Bedeutung, wo der zu Verfügung stehende Platz im Vergleich zu Anwendungen an der Lendenwirbelsäule deutlich geringer ist.

Ferner weist die Erfindung den Vorteil auf, dass ein elastisches Element wahlweise mit starren stabförmigen Elementen verschiedener Länge zu einem elastischen stabförmigen Element kombinierbar ist oder mit verschiedenen Schäften und/oder Köpfen zu einer Knochenschraube mit elastischen Eigenschaften kombinierbar ist. Das elastische stabförmige Element bzw. die Knochenschraube weisen dann in Abhängigkeit von dem verwendetem elastischen Element vorgegebene elastische Eigenschaften wie eine bestimmte Kompressions- und Extensionsfähigkeit in axialer Richtung, sowie eine bestimmte Biege- und Torsionssteifigkeit auf.

Insbesondere kann das elastisches Element mit stabförmigen Bauteilen verschiedener Dicke oder mit in der Wirbelsäulenund/oder Unfallchirurgie zu verwendenden Platten unterschiedlicher Form und Länge verbunden werden.

Weitere Merkmale und Zweckmäßigkeiten ergeben sich aus der Beschreibung von Ausführungsbeispielen anhand der Figuren.

Von den Figuren zeigen:
- Fig. 1: ein elastisches Element mit einer Doppelschraubenfeder nach einer ersten Ausführungsform der Erfindung;
- Fig. 2: eine schematische Darstellung der Doppelschraubenfeder des elastischen Elements aus Fig. 1 einmal als Ganzes (Fig. 2a) und in Explosionsdarstellung (Fig. 2b);
- Fig. 3: ein elastisches Element mit einer Doppelschraubenfeder nach einer zweiten Ausführungsform der Erfindung;
- Fig. 4a, 4b: ein elastisches Element mit einer Doppelschraubenfeder nach einer dritten Ausführungsform, einmal als Ganzes (Fig. 4a) und einmal in Explosionsdarstellung (Fig. 4b);
- Fig. 5a: ein elastisches Element mit einer Doppelschraubenfeder nach einer vierten Ausführungsform der Erfindung;
- Fig. 5b: eine Schnittdarstellung des elastischen Elements aus Fig. 5a;
- Fig. 6: eine Schnittdarstellung eines elastischen Elements mit einer Doppelschraubenfeder nach einer fünften Ausführungsform der Erfindung;
- Fig. 7a: ein elastisches Element mit einer Doppelschraubenfeder nach einer sechsten Ausführungsform der Erfindung;
- Fig. 7b: eine Schnittdarstellung des elastischen Elements aus Fig. 7a;
- Fig. 8a: ein stabförmiges Element mit dem erfindungsgemäßen elastischen Element;
- Fig. 8b: eine teilgeschnittene Explosionsdarstellung einer Polyxialknochenschraube mit dem elastischen Element;
- Fig. 8c: eine teilgeschnittene Darstellung einer Monoaxialschraube mit dem elastischen Element;
- Fig. 8d: eine Ansicht von oben auf einen aus einer Platte, dem elastischen Element und einen Stababschnitt bestehenden Teil einer Stabilisierungsvorrichtung für Knochen oder Wirbel, sowie einen Querschnitt durch die Platte;
- Fig. 9: eine erfindungsgemäß Stabilisierungseinrichtung für die Wirbelsäule;
- Fig. 10a-c: Schritte einer Ausführungsform eines Verfahrens zur Herstellung des elastischen Elementes; und
- Fig. 11: ein erfindungsgemäßes elastisches Element mit einem halbkreisförmigen Auslauf an beiden Enden der Doppelschraubenfeder.

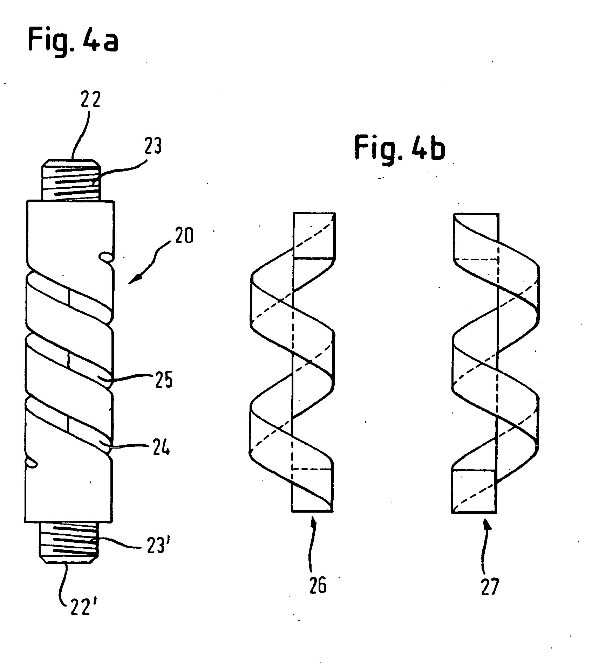

Die Figuren 4, 8b, 8c, 8d sowie 10a-c und 11 dienen lediglich der technischen Erläuterung.

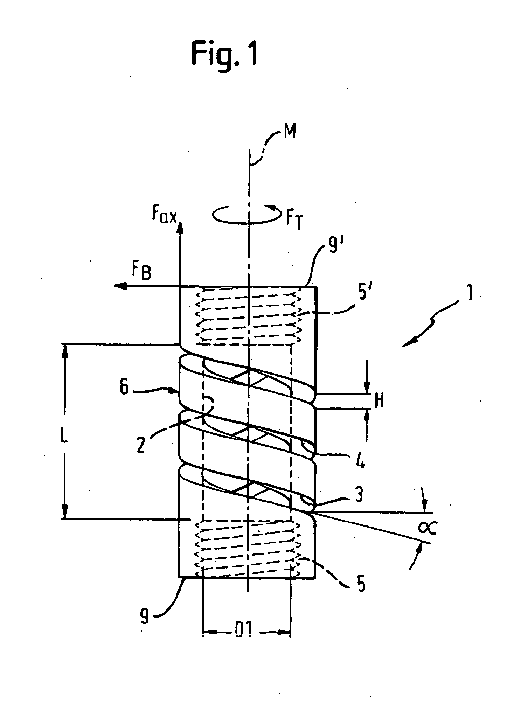

In Fig. 1 ist ein elastisches Element 1 nach einer ersten Ausführungsform dargestellt.

Das elastische Element 1 ist als hohlzylindrisches Element mit einer durchgehenden koaxialen Bohrung 2 und einer spiralförmig mit einer vorbestimmten Steigung und über eine vorbestimmte Länge in Richtung der Zylinderachse in der Wandung verlaufenden ersten Ausnehmung 3, die in radialer Richtung in die Bohrung 2 mündet, ausgebildet. Weiter ist spiralförmig mit der gleichen Steigung wie die erste Ausnehmung 3 über die gleiche Länge in Richtung der Zylinderachse M in der Wandung zwischen den Windungen der ersten Ausnehmung 3 eine zweite Ausnehmung 4 vorgesehen, die in radialer Richtung in die Bohrung 2 mündet. Dadurch ist eine Doppelschraubenfeder aus zwei Schraubenfedern gebildet, wobei die Windungen der einen Schraubenfeder zwischen den Windungen der zweiten Schraubenfeder verlaufen. Bevorzugt sind die Windungen der einen Schraubenfeder relativ zu den Windungen der anderen Schraubenfeder um ihre gemeinsame Mittenachse um 180° gedreht, sodass sich die erste und die zweite Ausnehmung genau gegenüberliegen.

Die Länge L der spiralförmigen Ausnehmungen 3, 4 in Richtung der Zylinderachse, die Höhe H der Ausnehmungen, die Steigung α der Schraubenlinien, entlang denen die Ausnehmungen 3, 4 ausgebildet sind, und der Durchmesser D1 der koaxialen Bohrung 2 sind so gewählt, dass eine gewünschte Steifigkeit des elastischen Elements gegenüber axialen Kräften Fₐₓ, Biegekräften F_{B} und Torsionskräften F_{T}, die auf das elastische Element wirken, gegeben ist. Angrenzend an seine beiden freien Enden weist das elastische Element 1 jeweils einen Abschnitt mit einem sich über eine vorbestimmte Länge erstreckenden Innengewinde 5, 5' auf. Die Innengewindeabschnitte überlappen in axialer Richtung nicht mit dem Abschnitt, in dem die Ausnehmungen in der Wandung ausgebildet sind. Der Außendurchmesser des elastischen Elements 1 ist der jeweiligen Anwendung entsprechend gewählt. Das elastische Element ist aus einem körperfreundlichen Material wie z.B. Titan ausgebildet.

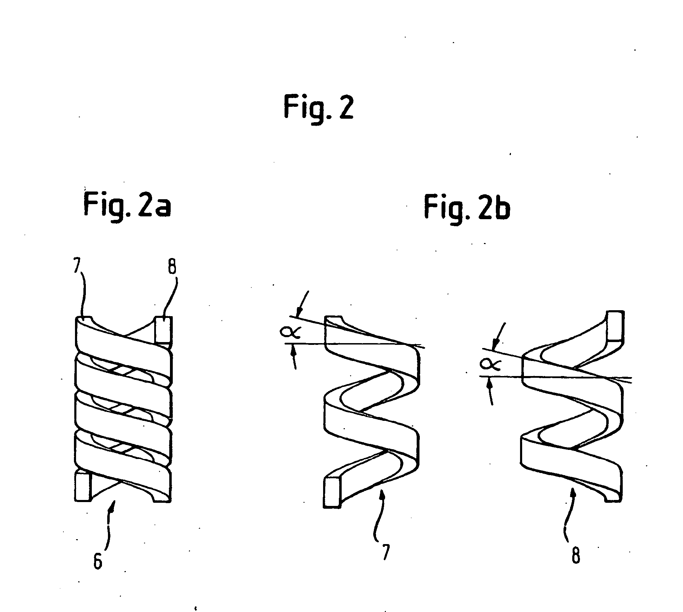

Fig. 2 veranschaulicht den Aufbau einer Doppelschraubenfeder 6 wie sie durch die Ausnehmungen 3, 4 in dem elastischen Element in Fig. 1 gebildet wird.

Die Doppelschraubenfeder 6 setzt sich zusammen aus zwei Schraubenfedern 7 und 8. Die beiden Schraubenfedern 7 und 8 sind identisch ausgebildet und besitzen insbesondere eine identische Steigung α, jedoch ist die erste Schraubenfeder 7 in der Doppelschraubenfeder 6 gegenüber der zweiten Schraubenfeder 8 um ihre gemeinsame Mittenachse um 180° gedreht. Die Windungen der ersten Schraubenfeder 7 verlaufen bei der Doppelschraubenfeder 6 daher in der Mitte zwischen den Windungen der zweiten Schraubenfeder 8 und umgekehrt.

Bei einer vorgegebenen Baulänge eines elastischen Elementes ist die Steigung der Schraubenfeder dadurch begrenzt, dass mindestens eine ganze Windung vorhanden sein muss, um gute Elastizitätseigenschaften zu erhalten. Bei einer Doppelschraubenfeder ist für jeden der Schraubenfedern weniger als eine ganze Windung notwendig, um gute Elastizitätseigenschaften zu erhalten. Daher kann bei gleicher Baulänge die Steigung der Schraubenfeder im Vergleich zu einer Einfachschraubenfeder erhöht werden. Eine Erhöhung der Steigung der Schraubenfeder ergibt bei gleicher Baulänge und gleichen sonstigen Eigenschaften eine Erhöhung der Biegesteifigkeit. Daher ist es mit einer Doppelschraubenfeder gegenüber einer Einfachschraubenfeder möglich bei gleichen Abmessungen eine höhere Biegesteifigkeit zu erreichen.

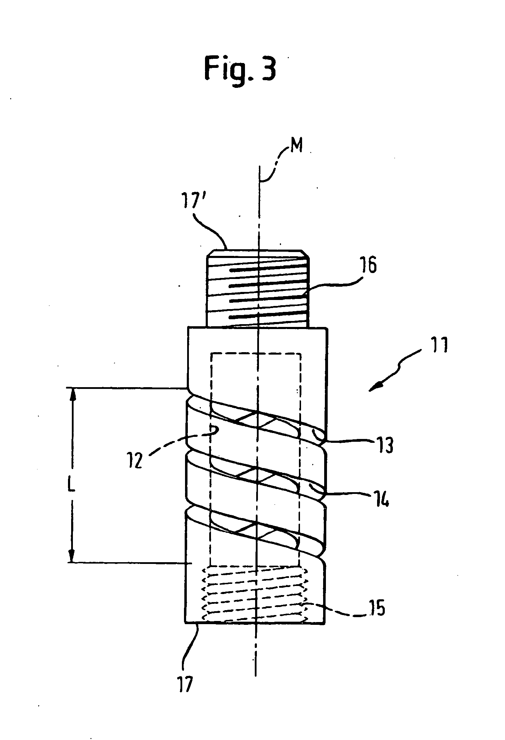

In Fig. 3 ist ein elastisches Element 11 nach einer zweiten Ausführungsform dargestellt.

Das elastische Element 11 nach einer zweiten Ausführungsform unterscheidet sich von dem elastischen Element 1 nach einer ersten Ausführungsform dadurch, dass anstelle einer sich von dem ersten bis zu dem zweiten Ende 17' des elastischen Elementes erstreckenden Bohrung 2 eine an das erste Ende 17 des elastischen Elements angrenzende Sackbohrung 12 vorgesehen ist. Die Sackbohrung erstreckt sich dabei über die gesamte Länge L der Doppelschraubenfeder, die wie bei der ersten Ausführungsform durch eine erste und eine zweite Ausnehmung 13 und 14 in der Wandung eines hohlzylindrischen Abschnittes gebildet wird. An das erste Ende 17 angrenzend ist in der Sackbohrung ein Innengewinde 15 vorgesehen. An dem dem ersten Ende 17 gegenüberliegenden zweiten Ende 17' weist das elastische Element einen zylinderförmigen Ansatz 16 mit einem Außengewinde auf.

In Fig. 4 ist ein elastisches Element nach einer dritten Ausführungsform dargestellt.

Das elastische Element 20 nach der dritten Ausführungsform unterscheidet sich dadurch von den anderen Ausführungsformen, dass keine Bohrung koaxial zur Mittenachse M des elastischen Elementes 20 vorgesehen ist. Des weiteren ist sowohl an das erste Ende 22, als auch an das zweite Ende 22' angrenzend ein zylindrischer Ansatz 23, 23' mit einem Außengewinde vorgesehen. Wie bei den zuvor beschriebenen Ausführungsformen werden durch zwei Ausnehmungen 24, 25 zwei Schraubenfedern 26, 27 gebildet .

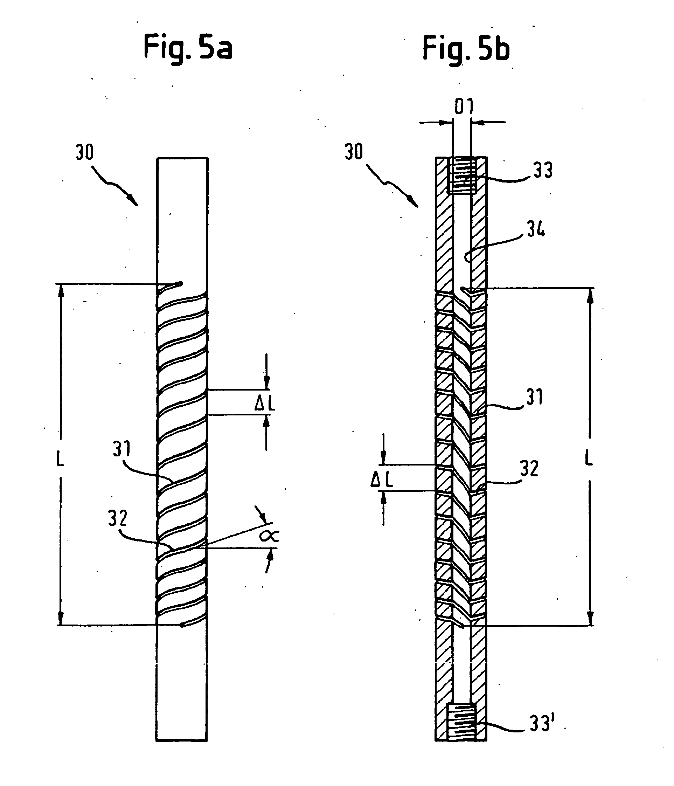

In Fig. 5a ist ein elastisches Element nach einer vierten Ausführungsform dargestellt. Fig. 5b ist eine Schnittdarstellung des elastischen Elements aus Fig. 5a.

Das elastische Element 30 nach der vierten Ausführungsform unterscheidet sich von dem nach der ersten Ausführungsform dadurch, dass die Steigung α der die Doppelschraubenfeder bildenden Ausnehmungen 31, 32 nicht konstant ist, sondern über die Länge L der Doppelschraubenfeder des elastischen Elements 30 variiert. Dabei variiert die Steigung α derart, dass der Abstand ΔL der Ausnehmungen 31, 32 von den freien Enden des elastischen Elements 30 aus gesehen zur Mitte hin zunimmt. Dementsprechend variiert auch die Biegesteifigkeit des elastische Elements 30, die mit zunehmendem Abstand ΔL der Ausnehmungen 31, 32 zunimmt. Über die Steigung der Ausnehmungen entlang der Mittenachse des elastischen Elements kann somit gezielt eine bestimmte ortsabhängige Biegesteifigkeit eingestellt werden.

Wie die erste Ausführungsform weist das elastische Element 30 nach der vierten Ausführungsform angrenzend an seine beiden freien Enden jeweils einen Abschnitt mit einem sich über eine vorbestimmte Länge erstreckenden Innengewinde 33, 33' und eine durchgehende koaxiale Bohrung 34 mit einem Innendurchmesser D1 auf.

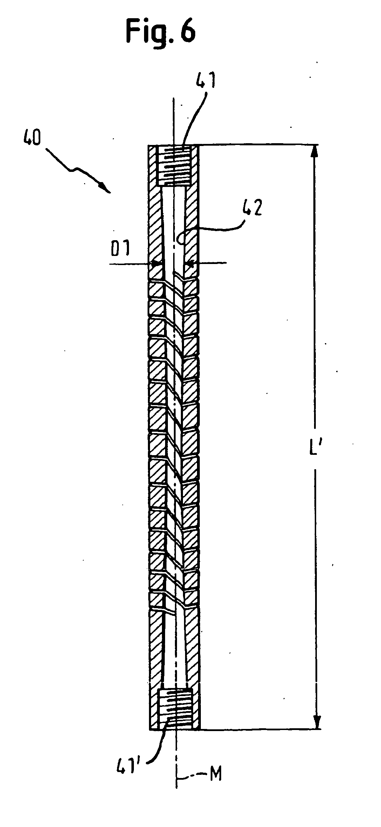

Fig. 6 ist eine Schnittdarstellung eines elastischen Elements nach einer fünften Ausführungsform.

Das elastische Element 40 nach der fünften Ausführungsform unterscheidet sich von dem elastischen Element 30 nach der vierten Ausführungsform dadurch, dass der Innendurchmesser D1 der durchgehenden koaxialen Bohrung 42 nicht konstant ist, sondern über die Länge L' des elastischen Elements 40 variiert. Der Innendurchmesser D1 der Bohrung 42 variiert dabei derart, dass er von den freien Enden aus gesehen zur Mitte des elastischen Elements 40 hin abnimmt. Dementsprechend variiert auch die Biegesteifigkeit des elastischen Elements 40, die mit abnehmendem Innendurchmesser D1 zunimmt. Über den Innendurchmesser der koaxialen Bohrung kann somit gezielt eine bestimmte ortsabhängige Biegesteifigkeit des elastischen Elements 40 eingestellt werden.

Wie die vierte Ausführungsform weist das elastische Element 40 angrenzend an seine beiden freien Enden jeweils einen Abschnitt mit einem sich über eine vorbestimmte Länge erstreckenden Innengewinde 41, 41'.

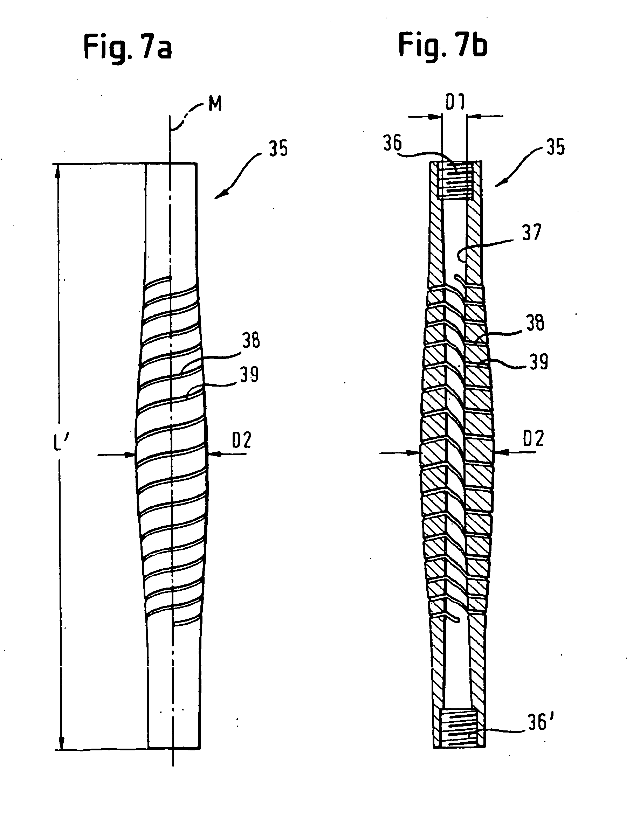

In Fig. 7a ist ein elastisches Element nach einer sechsten Ausführungsform dargestellt. Fig. 7b ist eine Schnittdarstellung des elastischen Elements aus Fig. 7a.

Das elastische Element 35 nach der sechsten Ausführungsform unterscheidet sich von dem nach der fünften Ausführungsform dadurch, dass der Außendurchmesser D2 des elastischen Elements 35 nicht konstant ist, sondern über die Länge L' de des elastischen Elements 35 variiert. Dabei variiert der Außendurchmesser D2 derart, dass er von den freien Enden aus gesehen zur Mitte des elastischen Elements 35 hin zunimmt. Dementsprechend variiert auch die Biegesteifigkeit des elastischen Elements 35, die mit zunehmendem Außendurchmesser des elastischen Elements zunimmt. Über den Außendurchmesser des elastischen Elements kann somit gezielt eine bestimmte ortsabhängige Biegesteifigkeit eingestellt werden.

Wie die vierte Ausführungsform weist das elastische Element 35 angrenzend an seine beiden freien Enden jeweils einen Abschnitt mit einem sich über eine vorbestimmte Länge erstreckenden Innengewinde 36, 36', eine durchgehende koaxiale Bohrung 37 mit einem Innendurchmesser D1 sowie zwei Ausnehmungen 38 und 39 in der Form einer Schraubenfeder auf, durch die eine Doppelschraubenfeder gebildet wird.

Abwandlungen des elastischen Elementes von den zuvor beschriebenen Ausführungsformen sind möglich.

So kann das elastische Element mehr als zwei Schraubenfedern beinhalten, wobei die Windungen einer Schraubenfeder jeweils zwischen den Windungen der anderen Schraubenfedern verlaufen.

In einer weiteren Abwandlung der zweiten Ausführungsform ist anstelle der Sackbohrung 12 eine sich über die gesamte Länge des elastischen Elementes erstreckende Bohrung vorgesehen, deren Durchmesser kleiner als der Außendurchmesser des zylindrischen Ansatzes 16 ist.

Die vierte bis sechste Ausführungsform wurde jeweils so beschrieben, dass die Biegesteifigkeit des elastische Elements über die Länge L der Doppelschraubenfeder von den freien Enden aus gesehen zur Mitte des elastischen Elements hin zunimmt. Jedoch kann durch entsprechende Gestaltung der Steigung α der beiden Ausnehmungen, des Außendurchmessers D2 des elastischen Elements und des Innendurchmesser D1 der koaxialen Bohrung die Biegesteifigkeit mit einer beliebigen andere Ortsabhängigkeit über die Länge L der Doppelschraubenfeder bzw. die Lange L' des elastischen Elements eingestellt werden.

Alle Ausführungsformen wurden so beschrieben, dass das elastische Element die Form eines Zylinders bzw. Hohlzylinders aufweist, jedoch kann die äußere Form auch von der exakten Form eines Zylinders abweichen und z.B. eine ovale Querschnittsfläche haben oder tailliert ausgebildet sein. Das elastische Element hat damit eine richtungsabhängige Biegeelastizität.

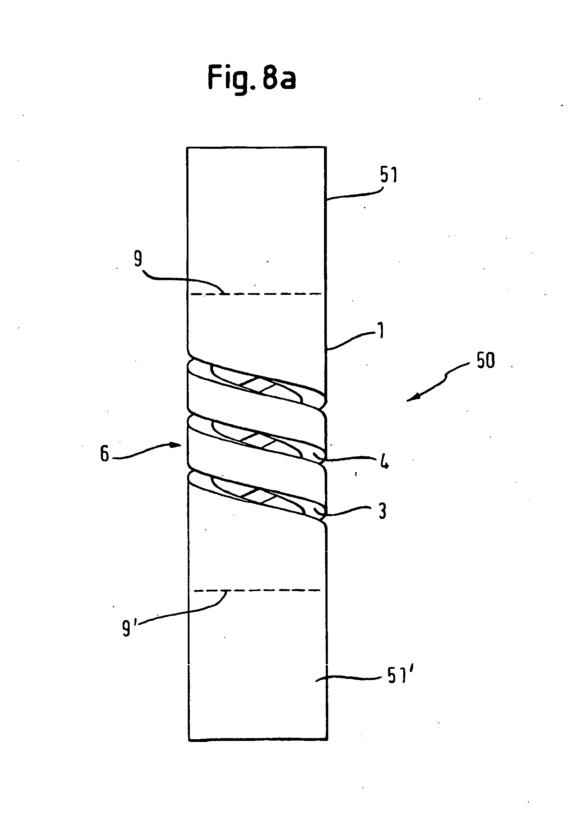

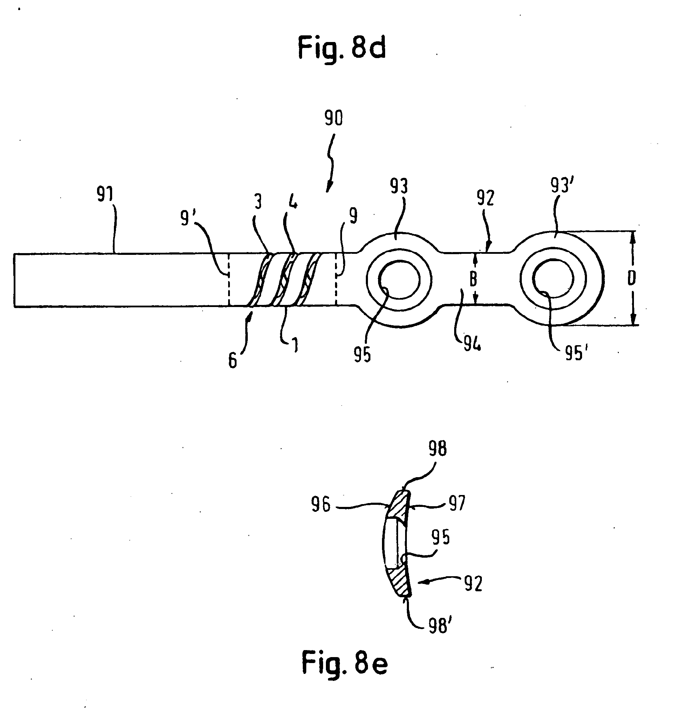

In einem ersten, in Fig. 8a gezeigten Beispiel gemäß der Erfindung ist das elastische Element 51 Bestandteil eines elastischen stabförmigen Elements 50. Das elastische stabförmige Element 50 besteht aus dem Federelement 1 und zwei zylindrischen Stabschnitten 51, 51', die an ihrem Ende jeweils einen nicht dargestellten zylindrischen Ansatz mit einem Außengewinde aufweisen, das jeweils mit einem Innengewinde 5 bzw. 5' des Federelements 1 zusammenwirkt. Die Stababschnitte 51, 51' und das Federelement 1 weisen in diesem Ausführungsbeispiel im wesentlichen denselben Außendurchmesser auf. Die Länge der Stababschnitte 51, 51' und des Federelements 1 sind unabhängig voneinander im Hinblick auf eine gewünschte Anwendung wählbar. Das elastische stabförmige Element 50 dient beispielsweise zur Verbindung von Pedikelschrauben an der Wirbelsäule. Das so gebildete stabförmige Element 30 nimmt durch die elastischen Eigenschaften des Federelementes 1 in vorbestimmten Umfang Kompressions-, Extensions-, Biege- und Torsionskräfte auf.

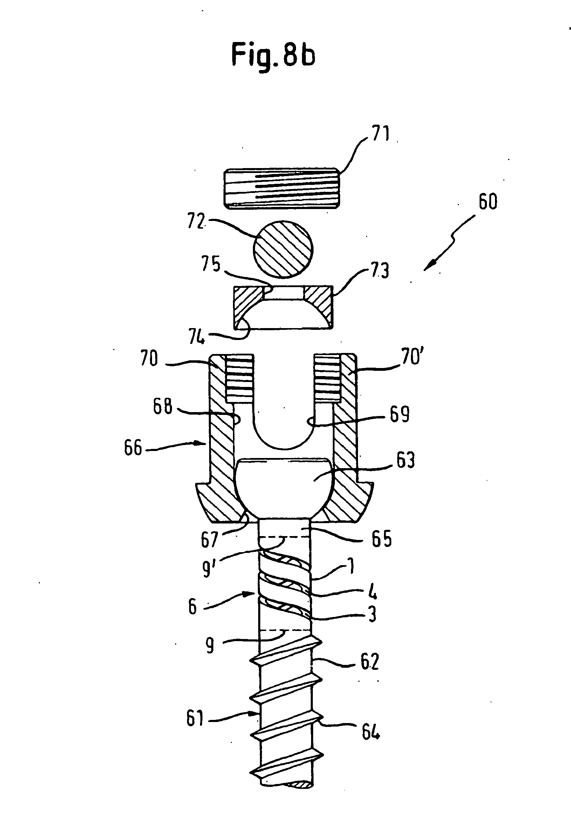

Fig. 8b zeigt ein zweites Beispiel des elastischen Elementes 1. Das elastischen Element 1 ist hier Bestandteil eines Knochenverankerungselements, das als PolyaxialKnochenschraube 60 ausgebildet ist.

Die Polyaxialknochenschraube 60 weist ein Schraubenelement 61 auf, welches aus dem elastischen Element 1, einem Gewindeschaft 62 in diesem Ausführungsbeispiel mit einer nicht dargestellten Spitze und einem Schraubenkopf 63 besteht.Der Gewindeschaft 62 weist ein Knochengewinde 64 zum Einschrauben in den Knochen und einen nicht dargestellten zylinderförmigen Ansatz mit einem Außengewinde auf, das mit dem Innengewinde 5 des Federelementes 1 zusammenwirkt. Der Schraubenkopf 63 weist einen zylinderförmigen Abschnitt 65 und daran angrenzend wie der Gewindeschaft 62 einen nicht dargestellten zylinderförmigen Ansatz mit einem Außengewinde auf, das mit dem Innengewinde 5' des Federelementes 1 zusammenwirkt.

Das Schraubenelement 61 ist in einem Aufnahmeteil 66 in unbelastetem Zustand schwenkbar gehalten. Das Aufnahmeteil 66 ist im wesentlichen zylindrisch ausgebildet und weist an seinem einen Ende eine axialsymmetrisch ausgerichtete erste Bohrung 67 auf, deren Durchmesser größer als der des Gewindeschafts 62 und kleiner als der des Schraubenkopfs 63 ist. Ferner weist das Aufnahmeteil 66 eine koaxiale zweite Bohrung 68 auf, die auf dem der ersten Bohrung 67 gegenüberliegenden Ende offen ist und deren Durchmesser so groß ist, dass das Schraubenelement 61 durch das offene Ende mit dem Gewindeschaft 62 durch die erste Bohrung 67 hindurchführbar ist, bis der Schraubenkopf 63 am Rand der ersten Bohrung 67 anliegt. Das Aufnahmeteil 66 weist eine sich vom freien Ende in Richtung der ersten Bohrung 67 erstreckende U-förmige Ausnehmung 69 auf, durch die zwei freie Schenkel 70, 70' gebildet sind. In einem Bereich angrenzend an ihr freies Ende weisen die Schenkel 70, 70' ein Innengewinde auf, welches mit einem entsprechenden Außengewinde einer Innenschraube 71 zum Fixieren eines Stabs 72 zusammenwirkt.

Es ist ferner ein Druckelement 73 zum Fixieren des Schraubenkopfs 63 in dem Aufnahmeteil 66 vorgesehen, das so ausgebildet ist, dass es an seiner dem Schraubenkopf 63 zugewandten Seite eine sphärische Ausnehmung 74 aufweist, deren Radius im wesentlichen gleich dem Radius des kugelsegmentförmigenAbschnitts des Schraubenkopfes 63 ist. Der Außendurchmesser des Druckelements 73 ist so gewählt, dass es in dem Aufnahmeteil 66 zu dem Schraubenkopf 63 hin verschiebbar ist. Das Druckelement 73 weist ferner eine koaxiale Bohrung 75 für den Zugriff auf eine nicht dargestellte Ausnehmung in dem Schraubenkopf 63 zum Ineingriffbringen mit einem Einschraubwerkzeug auf.

Im Betrieb wird in das Innengewinde 5 des elastischen Elementes 1 der Gewindeschaft 62 mit seinem nicht dargestellten zylinderförmigen Ansatz und in das Innengewinde 5' der Schraubenkopf 63 mit seinem nicht dargestellten zylinderförmigen Ansatz eingeschraubt, um das Schraubenelement 61 zu bilden. Danach wird das so gebildete Schraubenelement 61 mit dem Gewindeschaft 62 voran durch die zweite Bohrung 68 in das Aufnahmeteil 66 eingeführt, bis der Schraubenkopf 63 am Rand der ersten Bohrung 67 anliegt. Anschließend wird das Druckelement 73 mit der sphärischen Ausnehmung voran durch die zweite Bohrung 68 in das Aufnahmeteil 66 eingeführt. Dann wird das Schraubenelement 61 in den Knochen bzw. Wirbel eingeschraubt. Schließlich wird der Stab 72 in das Aufnahmeteil 66 zwischen die beiden freien Schenkel 70, 70' eingelegt, die Winkelstellung des Aufnahmeteils relativ zu dem Schraubenelement justiert und mit der Innenschraube 71 fixiert. Durch das elastische Element werden Bewegungen um die Ruhelage in begrenzter Weise ermöglicht.

Wenn das elastische Element 1 wenigstens teilweise mit seinem durch die Schraubenfedern gebildeten elastischen Abschnitt über die Knochenoberfläche hervorsteht kann das Federelement 1 Biegekräfte, sowie Zug- und Druckkräfte aufnehmen. Steht das elastische Element mit seinem durch die Schraubenfedern gebildeten elastischen Abschnitt nicht mehr über die Knochenoberfläche hervor, kann das Schraubenelement 61 trotzdem bei einer Bewegung des Knochens bzw. Wirbels etwas nachgeben. Damit wird verhindert, dass ungünstige Spannungen auftreten.

Die Polyaxialschraube ist nicht auf die zuvor beschriebene Ausführungsform beschränkt, sondern kann auch jede beliebige andere Polyaxialschraube mit einem wie oben beschriebenen dreiteiligen Schraubenelement sein.

Die Stabfixierung ist nicht auf die in Fig. 8b gezeigte Innenschraube beschränkt, sondern es kann zusätzlich eine Außenmutter vorgesehen sein, oder jede bekannte Art der Stabfixierung kann eingesetzt werden.

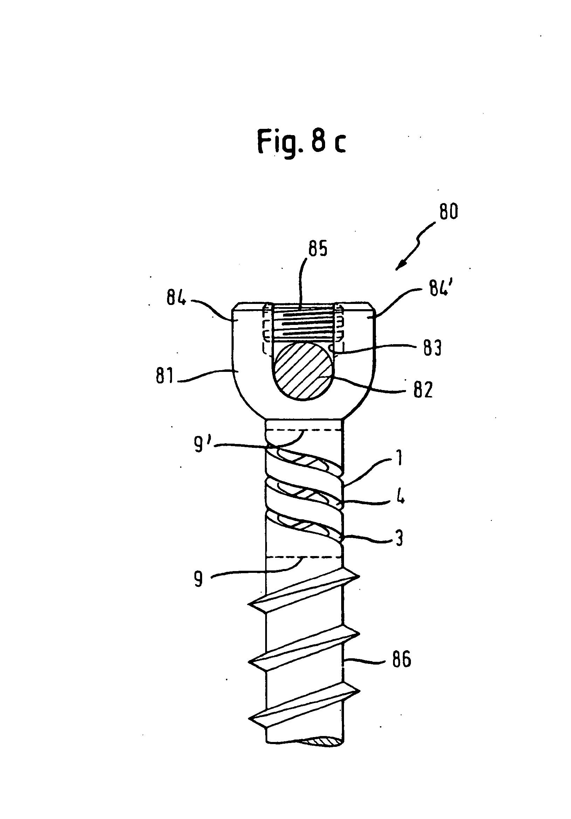

Als ein drittes Beispiel des elastischen Elements ist in Fig. 8c ein Knochenverankerungselement dargestellt, das als Monoaxialschraube 80 ausgebildet ist.

Bei der Monoaxialschraube 80 ist der Schraubenkopf als Aufnahmeteil 81 ausgebildet. Zur Aufnahme des Stabes 82 ist an das erste freie Ende des Aufnahmeteils 81 angrenzend eine U-förmige Ausnehmung 83 vorgesehen. Die durch die U-förmige Ausnehmung gebildeten freien Schenkel 84, 84'.weisen auf ihrer Innenseite ein Innengewinde auf, in das das Aussengewinde einer Innenschraube 85 eingreift. Der Stab 82 wird im zusammengebauten Zustand der Monoaxialschraube zwischen dem Boden der U-förmigen Ausnehmung 83 und der Innenschraube 85 festgeklemmt. Angrenzend an ein dem ersten freien Ende gegenüberliegenden zweiten Ende des Aufnahmeteils 81 ist ein nicht dargestellter zylindrischer Ansatz mit einem Außengewinde vorgesehen, der in das an das erste Ende des elastischen Elementes 1 angrenzende Innengewinde 5 eingreift. Angrenzend an das dem ersten Ende des elastischen Abschnittes 1 gegenüberliegende Ende weist die Monoaxialschraube einen Gewindeschaft 86 auf, der wie der oben beschriebene Gewindeschaft 62 der Polyaxialknochenschraube 60 ausgebildet ist und mit einem nicht dargestellten zylindrischen Ansatz mit einem Außengewinde in das Innengewinde 5' des elastischen Elements 1 eingreift.

Im Betrieb werden zunächst der Gewindeschaft 86 und das Aufnahmeteil in die beiden Innengewinde 5, 5' des elastischen Elements 1 eingeschraubt. Anschließend wird die Monoaxialschraube 80 in den Knochen oder den Wirbel eingeschraubt. Dann wird die U-förmige Ausnehmung 83 ausgerichtet und der Stab 82 eingelegt. Schließlich wird der Stab 82 mit der Innenschraube 85 fixiert.

Als weiteres Beispiel für das elastische Element 1 ist in Fig. 8d eine Draufsicht auf ein Verbindungselement 90 zu sehen, das aus einem stabförmigen Abschnitt 91, einem Federelement 1 und einer Platte 92 besteht.

Der stabförmige Abschnitt 91 weist einen nicht dargestellten zylindrischen Ansatz mit einem Außengewinde zum Einschrauben in das an das eine Ende des Federelementes 1 angrenzende Innengewinde 5 auf. Ebenso weist die Platte 92 einen nicht dargestellten zylindrischen Ansatz mit einem Außengewinde zum Einschrauben in das an das andere Ende des Federelementes 1 angrenzende Innengewinde 5' auf.

Die Platte besteht aus zwei in der Draufsicht kreisförmigen Abschnitten 93, 93', die über einen Stegabschnitt 94 miteinander verbunden sind. Die Breite B des Stegabschnittes 94 ist geringer als der Durchmesser D der kreisförmigen Abschnitte 93, 93'. Koaxial zu den kreisförmigen Abschnitten sind zwei Bohrungen 95, 95' für Senkschrauben durch die Platte vorgesehen.

Wie in Fig. 8d zu sehen weist die erste Seite 96 der Platte eine konvexe Krümmung auf während die zweite Seite 97 der Platte eine konkave Krümmung zum Anliegen dieser Seite an einen Knochen aufweist. Durch die unterschiedlichen Krümmungsradien der beiden Seiten 96, 97 der Platte 92 verjüngt sich die Platte 92 zu den seitlichen Rändern 98, 98' hin. Dadurch kann die Platte stabil und gleichzeitig raumsparend sein. Die Bohrungen 95, 95' sind in ihrer Form zur Aufnahme von Senkschrauben angepasst.

Abwandlungen der mit den Figuren 8a bis 8d beschriebenen Beispiele sind möglich. So wurde das stabförmige Element 50, die Polyaxialknochenschraube 60, die Monoaxialschraube 80 und das Verbindungselement 90 so beschrieben, dass das elastische Element 1 als separates Teil ausgebildet ist und mit den übrigen Teilen verschraubt ist. Es ist jedoch auch möglich, dass das elastische Element als Abschnitt eines einstückigen Schraubenelementes, einer einstückigen Monoaxialschraube und eines einstückigen Verbindungselementes mit einem Stab- und einem Plattenabschnitt ausgebildet ist. Weiter ist es auch möglich das elastische Element mit Passsitz mit den anderen Elementen wie dem Gewindeschaft 62, dem Stababschnitt 51, 51', mit der Platte 92 oder dem Schraubenkopf 63 zu verbinden.

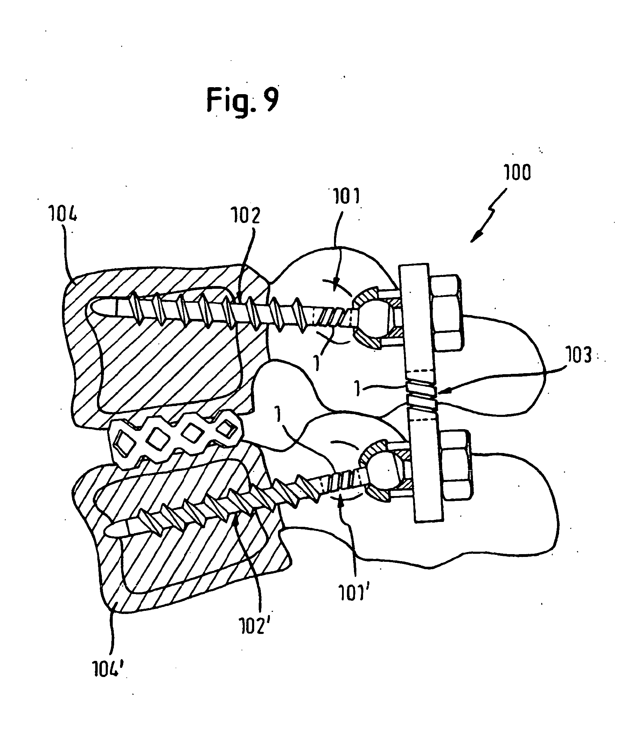

In Fig. 9 ist eine Stabilisierungsvorrichtung 100 für die Wirbelsäule dargestellt, wobei zwei Knochenverankerungselemente 101, 101' und ein diese verbindendes elastisches stabförmiges Element 103 vorgesehen ist. Die Schraubenelemente 102, 102' der Knochenverankerungselemente 101 bzw. 101' und das elastische stabförmige Element 103 weisen jeweils ein erfindungsgemäßes elastisches Element 1 auf. Die beiden Schraubenelemente 102, 102' sind in Wirbel 103, 103' eingeschraubt, sodass zwischen diesen Wirbeln über die Stabilisierungsvorrichtung 100 eine dynamische Stabilisierung hergestellt wird.

Durch die Mehrteiligkeit des elastischen stabförmigen Elementes und der Schraubenelemente ist es möglich, durch die Kombination von nur wenigen Grundelementen Stabilisierungsvorrichtungen 100 mit verschiedenen Eigenschaften zu erhalten. Die Stabilisierungsvorrichtung muß nicht notwendigerweise Knochenverankerungselemente mit einem elastischen Element und ein stabförmiges Element mit dem elastischen Element beinhalten. Je nach Anwendungsgebiet ist es auch möglich, nur ein stabförmiges Element mit einem elastischem Element und Knochenverankerungselemente ohne elastisches Element mit starren Schraubenelementen vorzusehen.

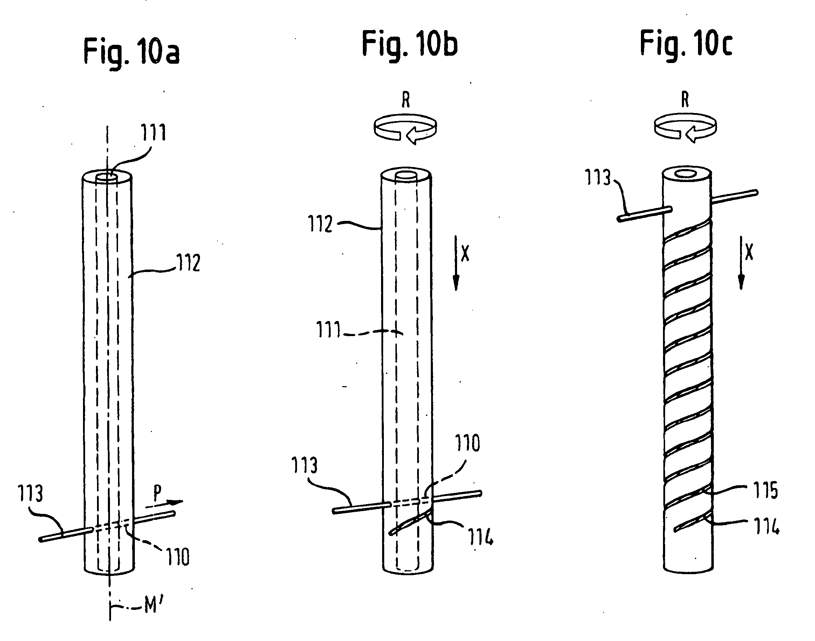

Die Herstellung eines elastischen Elementes 1 nach der ersten Ausführungsform ist in den Figuren 10a, 10b, und 10c dargestellt.

Zur Herstellung eines elastischen Elements 1 mittels Drahterodieren wird in einem Vollzylinder aus einem körpervertreglichen Material, wie z. B. Titan, senkrecht durch die Mittenachse des Zylinders eine sich durch den gesamten Zylinder erstreckende erste Bohrung 110 erzeugt. Dann wird eine zweite Bohrung 111 koaxial zu der Mittenachse des Zylinders über dessen gesamte Länge gebildet, sodass ein Hohlzylinder 112 entsteht. Die Reihenfolge des Bildens der ersten und der zweiten Bohrung ist beliebig und kann auch umgekehrt sein. Anschließend wird durch die erste Bohrung 110 zum Drahterodieren ein Draht 113 durchgeführt. Fig. 10a deutet diesen Verfahrensschritt durch einen Pfeil P an.

Im nächsten Schritt wird mit dem Draht 113 Drahterodieren durchgeführt, während der Hohlzylinder 112 entlang der Mittenachse in Richtung X mit konstanter Vorschubgeschwindigkeit relativ zum Draht verschoben und gleichzeitig mit konstanter Winkelgeschwindigkeit um seine Mittenachse gedreht wird. Die Drehung ist in Fig. 10b durch einen Pfeil D angedeutet. Dabei kommt es allein auf eine Relativbewegung zwischen Draht und Hohlzylinder an. So kann entweder der Draht raumfest sein und der Hohlzylinder bewegt werden oder aber auch umgekehrt. Auf diese Art und Weise werden durch Drahterodieren gleichzeitig zwei Schraubenlinien mit gleicher Steigung und zwei Ausnehmungen 114, 115 in der Wandung des Hohlzylinders gebildet, die in radialer Richtung in die Bohrung 111 münden. Fig. 10c zeigt das elastische Element kurz vor dem Ende des Schrittes des Drahterodierens. Nachdem die Ausnehmungen in axialer Richtung über eine vorbestimmte Länge gebildet worden sind, wird der Vorschub und die Drehung des Hohlzylinders gestoppt.

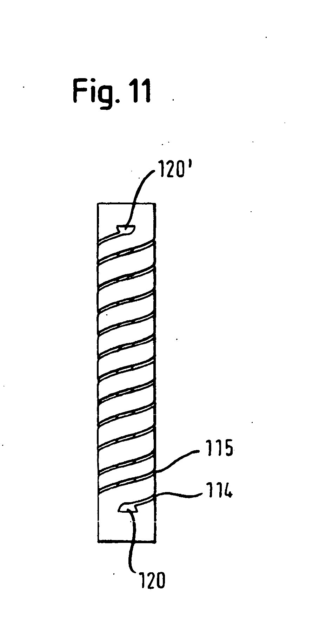

Wie in Fig. 11 dargestellt können zu Beginn des Drahterodierens und am Ende des Drahterodierens jeweils ein halbkreisförmiger Auslauf 120, 120' gebildet werde. Die Form des Auslaufs 120 bzw. 120' hat nicht zwingend die Form eines Halbkreises, sondern kann auch jede andere beliebige Form, wie die eines anderen Kreisabschnittes, haben, durch die Belastungsspitzen in dem Material am Übergang vom flexiblen Abschnitt zum festen Abschnitt im Betrieb gering gehalten werden.

Der Vorteil des oben beschriebenen Verfahrens mit Drahterodieren liegt für die Erzeugung des Auslaufs darin, dass die Ausläufe der beiden Schraubenfedern jeweils in einem gemeinsamen Arbeitsschritt hergestellt werden können, ein zusätzliches Umschalten zwischen den Achsen der Drahterodiermaschine ist bei dieser Ausführungsform im Vergleich zur Herstellung einer Einfachschraubenfeder nicht notwendig.

Schließlich wird in den zwei an die beiden Enden angrenzenden Endabschnitten der koaxialen Bohrung entlang der Mittenachse jeweils ein Innengewinde 5, 5' gebildet.

Alternativ kann das elastische Element 1 auch mittels Fräsen hergestellt werde. Dabei geht man wiederum von einem Zylinder mit einem vorbestimmten Außendurchmesser aus einem körperverträglichen Material, wie z.B. Titan, aus und fräst mit einem dünnen Scheibenfräser entlang einer ersten Schraubenlinie, deren Hauptachse kollinear zu der Hauptachse des Zylinders ist eine erste Ausnehmung. Dann wird in einem zweiten Schritt entlang einer zweiten Schraubenlinie, deren Windungen zwischen den Windungen der ersten Schraubenlinie verlaufen eine weitere Ausnehmung gebildet. Anschließend wird entlang der Hauptachse des Zylinders über die gesamte Länge des Zylinders eine Bohrung derart gebildet, dass die Ausnehmungen in diese Bohrung münden. Der Auslauf der Spirale am Übergang zwischen dem Spiralabschnitt und dem endseitigen Abschnitt des elastischen Elementes hat einen großen Einfluss auf die Stabilität des elastischen Elements 1. Daher wird mit einem Fingerfräser der Auslauf der Spirale an beiden Enden der Spirale derart nachbearbeitet, dass die scharfe Kante an der Innenseite der Bohrung entfernt wird. Dazu wird der Auslauf mit einem Fingerfräser in einem Winkel tangential zur Spiralkontur gefräst. Im Anschluss daran wird das Bauteil innen und außen entgratet.

Schließlich wird wie bei dem zuvor beschriebenen Verfahren in den zwei Endabschnitten der koaxialen Bohrung entlang der Mittenachse jeweils ein Innengewinde 5, 5' gebildet.

Weitere alternative Herstellungsverfahren für das elastische Element sind die Laserbearbeitung oder die Wasserstrahlbearbeitung, wobei die Herstellung analog dem Drahterodieren erfolgt, jedoch anstelle des gleichzeitigen Bildens von zwei Ausnehmungen durch einen durch die erste Bohrung durchgeführten Draht durch einen durch die erste Bohrung geführten Laserstrahl oder Wasserstrahl erfolgt.

In einer Abwandlung wird bei den obigen Verfahren anstelle zumindest eines der Innengewinde 5, 5' zu Beginn des Verfahrens durch Drehen ein zylinderförmiger Ansatz mit einem Außengewinde gebildet. In diesem Fall muss der Durchmesser der Bohrung 111 kleiner als der Durchmesser des zylinderförmigen Ansatzes sein.

In einer weiteren Abwandlung des Herstellungsverfahrens wird das Federelement entsprechend der zweiten und dritten Ausführungsform ohne eine durchgehende Bohrung 111 hergestellt.

## Patentansprüche

1. Stabilisierungseinrichtung zur dynamischen Stabilisierung der Wirbelsäule mit
zumindest zwei Knochenverankerungselementen, die in Wirbel einzuschrauben sind,
einem stabförmigen Element (103), welches die Knochenverankerungselemente verbindet,
wobei ein Abschnitt des stabförmigen Elements als elastisches Element ausgebildet ist, welches als ein im Wesentlichen zylinderförmiger Körper mit einem ersten Ende (9, 17, 22) und einem diesem gegenüberliegenden zweiten Ende (9', 17',22') und mit einem elastischen Abschnitt zwischen dem ersten und dem zweiten Ende ausgebildet ist und wobei in dem elastischen Element koaxial zur Mittenachse (M) eine durchgehende Bohrung (2, 34, 37, 42), die sich von dem ersten Ende (9) bis zu dem zweiten Ende (9') des zylinderförmigen Körpers erstreckt, oder eine sich an das erste Ende angrenzende Sackbohrung vorgesehen ist, und wobei
der elastische Abschnitt aus wenigstens zwei Schraubenfedern ausgebildet ist, die koaxial derart angeordnet sind, dass die Windungen einer Schraubenfeder (7) zumindest in einem Abschnitt der Schraubenfeder zwischen den Windungen einer anderen Schraubenfeder (8) verlaufen und wobei jede der beiden im wesentlichen gleichen Schraubenfedern (7, 8) relativ zur anderen um ihre gemeinsame Mittenachse um 180° gedreht ist und
wobei die Schraubenfedern durch eine spiralförmig in der Wandung des zylinderförmigen Körpers verlaufende erste Ausnehmung (3) und eine spiralförmig zweite Ausnehmung (4) gebildet sind, die in radialer Richtung in die Bohrung (2) münden,
wobei das stabförmige Element mehrstückig ausgebildet ist und wenigstens einen starren Stababschnitt und das elastische Element als separates Element aufweist, das mit dem starren Stababschnitt an seinem einen Ende verbunden ist.

2. Stabilisierungseinrichtung nach Anspruch 1, wobei die Schraubenfedern den gleichen Außendurchmesser aufweisen.

3. Stabilisierungseinrichtung nach Anspruch 1 oder 2, wobei die Schraubenfedern (7,8) in einer Ebene, die die Mittenachse der Schraubenfedern (7,8) enthält, den gleichen Querschnitt der Windung (3,4) aufweisen.

4. Stabilisierungseinrichtung nach einem der Ansprüche 1 bis 3, wobei der Durchmesser (D1) der durchgehenden Bohrung (37,42) entlang der Mittenachse (M) variiert.

5. Stabilisierungseinrichtung nach einem der Ansprüche 1 bis 4, wobei
das elastische Element aus einem körperverträglichen Material, insbesondere Titan, besteht.

6. Stabilisierungseinrichtung nach einem der Ansprüche 1 bis 5, bei dem der Außendurchmesser (D2) des elastischen Elements entlang der Mittenachse (M) variiert.

7. Stabilisierungseinrichtung nach einem der Ansprüche 1 bis 6, bei dem die Steigung (α) der wenigstens zwei Schraubenfedern (31,32) entlang der Mittenachse (M) variiert.

8. Stabilisierungseinrichtung nach einem der Ansprüche 1 bis 7, wobei bei dem elastischen Element an das erste Ende angrenzend ein erster zylindrischer Ansatz (16, 23, 23') mit einem Außengewinde oder eine erste Bohrung mit einem Innengewinde (5, 15, 33, 36, 41) vorgesehen ist zum Verbinden mit einem Stabsabschnitt (51).

9. Stabilisierungseinrichtung nach einem der Ansprüche 1 bis 7, wobei bei dem elastischen Element
an das erste Ende (9) und das zweite Ende (9') angrenzend ein Innengewinde (5', 15',33',36',41') vorgesehen ist zum Verbinden mit einem Stababschnitt (51').

## Claims

1. Stabilization device for the dynamic stabilization of the vertebral column including:
at least two bone anchoring elements to be screwed into vertebrae,
a rod-shaped element (103), which connects the bone anchoring elements,
wherein a section of the rod-shaped element is formed as an elastic element, which is formed as a substantially cylindrical body having a first end (9, 17, 22) and a second end (9', 17', 22') opposite the first end and having an elastic section between the first and the second end, and wherein in the elastic element a continuous bore (2, 34, 37, 42) which is coaxial to the centre axis (M) and extends from the first end (9) to the second end (9') of the cylindrical body, or a blind hole disposed adjacent to the first end, is provided,
the elastic section is formed by at least two helical springs, which extend coaxially, such that the windings of one helical spring (7) are at least in a portion of the helical spring extend between the windings of the other helical spring (8), and wherein each of the two substantially similar helical springs (7, 8) are rotated with respect to each other about the common centre axis by 180°, and
wherein the helical springs are formed by a recess (3) extending helically in the wall of the cylindrical body and a second helical recess (4), which open in radial direction into the bore (2),
wherein the rod-shaped element is formed as a multi piece and has at least a rigid rod section and the elastic element as a separate member, which is connected with the rigid rod section at its one end.

2. Stabilization device according to claim 1, wherein the helical springs have a same outer diameter.

3. Stabilization device according to claim 1 or 2, wherein the helical springs (7, 8) have a same cross section of a winding (3, 4) in a plane, which comprises the centre axis of the helical springs (7, 8).

4. Stabilization device according to claims 1 to 3, wherein the diameter (D1) of the continuous bore (37, 42) varies along the centre axis (M).

5. Stabilization device according to claims 1 to 4, wherein
the elastic element contains a body compatible material , in particular titanium.

6. Stabilization device according to claims 1 to 5, wherein the outer diameter (D2) of the elastic element varies along the centre axis (M).

7. Stabilization device according to claims 1 to 6, wherein the lead (α) of the at least two helical springs (31, 32) varies along the centre axis (M).

8. Stabilization device according to claims 1 to 7, wherein the elastic element has adjacent its first end a first cylindrical boss (16, 23, 23') having an outer thread or a first bore having an inner thread (5, 15, 33, 36, 41) for connection with a rod section (51).

9. Stabilization device according to one of claim 1 to 7, wherein the elastic element has adjacent its first end (9) and its second end (9') an inner thread (5', 15', 33', 36', 41') for connection with a rod section (51').

## Revendications

1. Dispositif de stabilisation pour la stabilisation dynamique de la colonne vertébrale, comprenant :
au moins deux éléments d'ancrage à l'os, à visser dans des vertèbres,
un élément en forme de tige (103), reliant les éléments d'ancrage à l'os,
un tronçon de l'élément en forme de tige étant réalisé en tant qu'élément élastique, réalisé sous forme d'un corps sensiblement à forme cylindrique, avec une première extrémité (9, 17, 22) et une deuxième extrémité (9', 17', 22') opposée à celle-ci, et avec un tronçon élastique, entre la première et la deuxième extrémité, et un perçage (2, 34, 37, 42) continu, ménagé dans l'élément élastique, coaxialement à l'axe central (M), s'étendant de la première extrémité (9) à la deuxième extrémité (9') du corps cylindrique, ou un trou borgne, s'arrêtant à la première extrémité, étant prévu, et où
le tronçon élastique est composé d'au moins deux ressorts hélicoïdaux, disposés coaxialement de manière que les enroulements d'un ressort hélicoïdal (7), au moins dans un tronçon du ressort hélicoïdal, s'étendent entre les enroulements d'un autre ressort hélicoïdal (8), et où chacun des deux ressorts hélicoïdaux (7, 8) sensiblement identiques est tourné à 180° par rapport à leur axe central commun, et
les ressorts hélicoïdaux sont formés par un premier évidement (3), s'étendant en forme de spirale dans la paroi du corps à forme cylindrique, et un deuxième évidement (4) en forme de spirale, débouchant, en direction radiale, dans le perçage (2),
l'élément en forme de tige étant réalisé en plusieurs parties et présentant au moins un tronçon de tige rigide et l'élément élastique, réalisé sous forme d'élément séparé, relié au tronçon de tige rigide, à une de ses extrémités.

2. Dispositif de stabilisation selon la revendication 1, les ressorts hélicoïdaux présentant le même diamètre extérieur.

3. Dispositif de stabilisation selon la revendication 1 ou 2, les ressorts hélicoïdaux (7, 8), dans un plan contenant l'axe médian des ressorts hélicoïdaux (7, 8), présentant la même section transversale de l'enroulement (3, 4).

4. Dispositif de stabilisation selon l'une quelconque des revendications 1 à 3, le diamètre (D1) du perçage (37, 42) continu variant, en évoluant le long de l'axe central (M).

5. Dispositif de stabilisation selon l'une quelconque des revendications 1 à 4,
l'élément élastique étant composé d'un matériau physiologiquement compatible, en particulier de titane.

6. Dispositif de stabilisation selon l'une quelconque des revendications 1 à 5, dans lequel le diamètre extérieur (D2) de l'élément élastique varie, en évoluant le long de l'axe médian (M).

7. Dispositif de stabilisation selon l'une quelconque des revendications 1 à 6, dans lequel le pas (α) des au moins deux ressorts hélicoïdaux (31, 32) varie, en évoluant le long de l'axe médian (M).

8. Dispositif de stabilisation selon l'une quelconque des revendications 1 à 7, dans lequel, pour l'élément élastique, un premier appendice (16, 23, 23') cylindrique, attenant à la première extrémité, avec un filetage extérieur, ou un premier perçage avec un filetage intérieur (5, 15, 33, 36, 41), est prévu pour liaison à un tronçon de tige (51).

9. Dispositif de stabilisation selon l'une quelconque des revendications 1 à 7, dans lequel, pour l'élément élastique, attenant à la première extrémité (9) et à la deuxième extrémité (9'), un filetage intérieur (5', 15', 33', 36', 41'), est prévu pour liaison à un tronçon de tige (51').
